(19) Europäisches Patentamt
European Patent Office
Office européen des brevets

(11) EP 2 144 865 B1

(12) **EUROPÄISCHE PATENTSCHRIFT**

(45) Veröffentlichungstag und Bekanntmachung des Hinweises auf die Patenterteilung:
**13.07.2016 Patentblatt 2016/28**

(21) Anmeldenummer: **08749287.2**

(22) Anmeldetag: **02.05.2008**

(51) Int Cl.:
***C07C 67/29*** (2006.01) ***C07C 67/56*** (2006.01)
***C07C 69/54*** (2006.01) ***C08F 220/28*** (2006.01)
***C08F 220/30*** (2006.01) ***G02B 1/04*** (2006.01)

(86) Internationale Anmeldenummer:
**PCT/EP2008/003540**

(87) Internationale Veröffentlichungsnummer:
**WO 2008/138497 (20.11.2008 Gazette 2008/47)**

(54) **VERFAHREN ZUR HERSTELLUNG VON DIOLEN**
METHOD FOR PRODUCING DIOLS
PROCÉDÉ DE PRODUCTION DE DIOLS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **14.05.2007 DE 102007022521**

(43) Veröffentlichungstag der Anmeldung:
**20.01.2010 Patentblatt 2010/03**

(73) Patentinhaber: **Cognis IP Management GmbH**
**40589 Dusseldorf (DE)**

(72) Erfinder:
- **MCKENNA, Peter**
  **Manningtree, Suffolk CO11 1PB (GB)**
- **SMALLRIDGE, Mark**
  **Hythe SO45 6BD (GB)**
- **MATTHEWS, Melissa**
  **Southampton, Hampshire SO45 3HR (GB)**
- **ROBERTS, Simon**
  **Romsey, Hampshire S051 7RW (GB)**

(74) Vertreter: **BASF IP Association**
**BASF SE**
**ZRX-C6**
**67056 Ludwigshafen (DE)**

(56) Entgegenhaltungen:
**WO-A-00/63149 WO-A-00/63150**
**GB-A- 852 384 JP-A- 63 002 952**

- **ROLF KALTOFEN U. A.: "Tabellenbuch Chemie" 1988, VERLAG HARRI DEUTSCH , THUN - FRANKFURT/M. , XP002494494 Seite 171; Tabelle 12.2**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

## Gebiet der Erfindung

Die Erfindung betrifft ein Verfahren zur Herstellung von Diolen spezieller Struktur, die durch die unten angegebene Formel (I) gekennzeichnet sind. Dabei handelt es sich um Substanzen, die neben zwei OH-Gruppen eine C=C-Doppelbindung enthalten, so dass sie als Monomere zur Herstellung von Polymeren geeignet sind.

## Stand der Technik

Glycerinmonomethacrylat (glyceryl mono methacrylate), nachfolgend abkürzend als GMMA bezeichnet, kann durch Hydrolyse von Isopropylidenglycerinmethacrylat (isopropylideneglyceryl methacrylate), nachfolgend abkürzend als IPGMA bezeichnet, gewonnen werden.

GMMA hat die nachstehende Struktur (A):

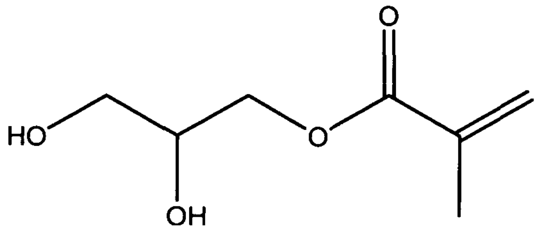

(A)

IPGMA hat die nachstehende Struktur (B). IPGMA wird in der Literatur bisweilen auch mit GMAK abgekürzt.

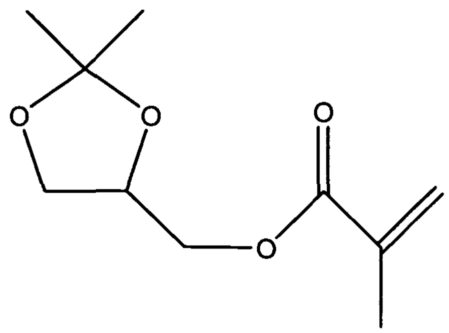

(B)

Gemäß üblicher Nomenklatur kann IPGMA auch als 2,2-Dimethyl-1,3-Dioxolan-4-yl-methylmethacrylat bezeichnet werden.

Bereits GB 852,384 **-** publiziert 1960 - beschreibt die Herstellung von GMMA durch Hydrolyse von IPGMA, wobei die Hydrolyse in Gegenwart verdünnter wäßriger Lösungen starker Mineralsäuren durchgeführt wird. Beispielhaft werden als geeignete Mineralsäuren Schwefelsäure, Salzsäure, Salpetersäure und Phosphorsäure (sulphuric acid, hydrochloric acid, nitric acid, phosphoric acid) genannt. Die typische Arbeitsweise geht aus Beispiel 1 hervor. Gemäß diesem Beispiel wurde im Anschluss an die Hydrolyse von IPGMA die im Reaktionsgemisch vorhandene Schwefelsäure durch Hinzufügen von Bariumhydroxid entfernt. Dabei bildete sich schwerlösliches Bariumsulfat, das durch Filtration abgetrennt wurde. Das rohe Zielprodukt GMMA wurde in etwa 20%-iger Lösung in Wasser/Aceton (12:1) erhalten. Sofern jedoch angestrebt war, das Zielprodukt zu isolieren, wurde die Lösung mit NaCl gesättigt, mit Benzol oder Ether extrahiert und das organische Lösungsmittel abgezogen.

Das aus GB 852,384 bekannte Verfahren hat jedoch gravierende Nachteile. So stellt der Einsatz von Bariumhydroxid wegen seines Schwermetallgehalts sowohl aus ökologischen als auch aus toxikologisches Gründen ein gewisses Risiko dar. Der zusätzliche Einsatz von organischen Lösungsmitteln ist ein weiterer unerwünschter Faktor. Auch in den Beispielen 2 bis 4 wird das brennbare und giftige Benzol als Lösungsmittel eingesetzt, wobei hier offenbar keine Neutralisation stattfindet und das Zielprodukt aus der sauren wässrigen Reaktionsmischung direkt mittels Benzol extrahiert wird. Diese Variante hat über die Verwendung von Benzol als Lösungsmittel hinaus den Nachteil, dass nicht

sichergestellt ist, dass Restspuren von Säure im Zielprodukt GMMA verbleiben. Selbst Spuren von Säure sind jedoch äußerst unerwünscht, weil sie erheblichen Einfluss auf die Stabilität von GMMA haben, indem sie dessen Stabilität vermindern.

Die Anmelderin hat darüber hinaus in eigenen Untersuchungen bei der Nacharbeitung von Beispiel 1 der genannten GB 852,384 folgendes festgestellt: Neutralisiert man die Reaktionsmischung mit Bariumhydroxid und zieht das Wasser daraus ab (to strip) ohne eine Extraktion mit einem organischen Lösungsmittel anzuwenden, so erhält man ein GMMA mit einem hohen Gehalt an Barium (> 1000 ppm).

In Summe kann die in GB 852,384 offenbarte Methode nicht befriedigen, insbesondere dann nicht, wenn eine hohe GMMA-Qualität angestrebt wird.

Auch zwanzig Jahre nach der oben zitierten britischen Patentschrift ist noch kein entscheidender Durchbruch bei der Herstellung von GMMA zu verzeichnen. So ist US-A-4,056,496 **-** publiziert 1977 - in Spalte 4, Zeile 42ff ein Verweis auf GB 852,384 zu entnehmen und zwar auf die Hydrolyse und die dort offenbarten anschließenden Prozeduren. Dementsprechend setzt Beispiel 1 von US-A-4,056,496 zur Hydrolyse Schwefelsäure und zur Neutralisation Bariumhydroxid ein, gefolgt von Filtration, Zugabe von NaCl, Extraktion mit Benzol oder Ether und Abziehen des organischen Lösungsmittels.

Und selbst gut weitere 20 Jahre später wird Beispiel 1 von in US-A-6,011,081 immer noch auf die "alten Methoden" verwiesen, nämlich auf US-A-4,056,496.

Der Einsatz von GMMA hoher Reinheit kommt für verschiedene technische Anwendungsgebiete in Frage, z.B. wenn GMMA als Monomer für die Herstellung von Polymeren für Beschichtungszwecke, Lacken, Farben, Klebstoffe oder Kontaktlinsen eingesetzt werden soll. Insbesondere stellt GMMA bei der Herstellung von Kontaktlinsen ein technisch sehr bedeutsames Monomer dar. In dem hier in Rede stehenden Zusammenhang sei auf den in EP-B-1,171,410 B1 auf S. 2, Zeile 6 bis S. 3, Zeile 19 referierten Stand der Technik verwiesen.

Das Ziel, Monomere - wie etwa GMMA - mit hoher Reinheit bereitzustellen, ist vor allem deshalb sehr wichtig, weil selbst Verunreinigungen im Spurenbereich sich einerseits bei der Lagerung der Monomeren und andererseits bei der Produktqualität von daraus hergestellten Polymeren äußerst negativ bemerkbar machen können. Besonders unerwünscht sind solche Verunreinigungen in Monomeren, die bei einer Polymerisation als Vernetzer (cross-linkers) fungieren können, da die Anwesenheit von Vernetzern im Zuge einer Polymerisation die Bildung linearer Polymere verhindert und/oder inhibiert.

Die jüngeren Patentschriften EP-B-1,171,410 **B1** and EP-B-1,171,411 **B1** beschreiben die Herstellung von GMMA durch Hydrolyse von IPGMA, wobei es jeweils als erfindungswesentliches Merkmal anzusehen ist, dass die Hydrolyse von IPGMA in Gegenwart einer immobilisierten Säure durchgeführt wird. Ausweislich Beispiel 1 der EP-B-1,171,410 B1 werden in einem Reaktionsgefäß IPGMA, entionisiertes Wasser und ein Kationaustauscher vorgelegt und durch die Mischung 48 h ein Luftstrom geleitet, der für die Durchmischung des Reaktionsansatzes sorgt. Ein Nachteil des hier beschriebenen Verfahrens ist: Durch das Einblasen eines Luftstromes während der Hydrolyse erfährt der saure Ionentauscher einen erhöhten Abrieb, der bei hohen Ansprüchen an die Produktreinheit nicht tragbar und auch durch anschließende Filtration nicht vollständig entfernbar ist.

Im übrigen sind mehrere weitere Nachteile der durch die PCT-Parallelen zu den Patentschriften EP-B-1,171,410 B1 und EP-B-1,171,411 B1 gegebenen Stand der Technik (diese Parallelen sind WO 00/63149 und WO 00/63150) in der DE-A-103,49,972 genannt (vergleiche dort auf Seite 2, Abschnitt [0007] und Abschnitt [0009].

Die Technik der Hydrolyse in Gegenwart eines sauren Ionenaustauschers wird auch in der jüngeren Anmeldung DE-A-103,49,972 A1 beschrieben, wobei jedoch gemäß dem hier beschriebenen Verfahren zwingend eine kontinuierliche Arbeitsweise einzuhalten ist. Die Reaktion wird in einem Festbett durchgeführt und die durch Hydrolyse freigesetzte Carbonylverbindung kontinuierlich aus dem Reaktionsmedium entfernt.

## Beschreibung der Erfindung

Aufgabe der vorliegenden Erfindung war es, ein Verfahren zur Herstellung von polymerisierbaren Monomeren der untenstehenden Formel (I) bereitzustellen, das nicht die Nachteile des bekannten Standes der Technik aufweist. Das Verfahren sollte insbesondere zu Verbindungen (I) mit hoher Reinheit führen. Es sollte sich insbesondere zur Herstellung von GMMA aus IPGMA eignen. Die Verbindungen (I) sollten sich zur Herstellung von Kontaktlinsen eignen. Das Verfahren sollte daher insbesondere zu Verbindungen (I) mit sehr geringem Gehalt an aciden Verbindungen führen, was wichtig ist, weil es die Wahrscheinlichkeit von Ablagerungen an Proteinen und Lipiden auf Kontaktlinsen reduziert.

Gegenstand der vorliegenden Erfindung ist ein Verfahren zur Herstellung von polymerisierbaren Monomeren der Formel (I)

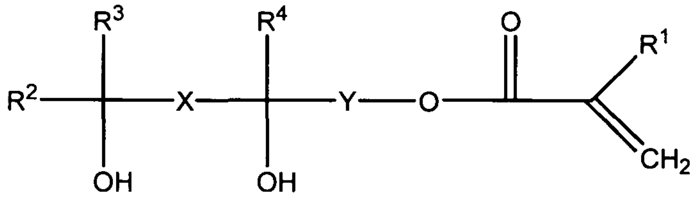

(I)

worin

- der Rest $R^1$ Wasserstoff oder eine Alkylgruppe mit 1 bis 4 C-Atomen,
- die Reste $R^2$ bis $R^4$ unabhängig voneinander Wasserstoff oder einen aliphatischen, cycloaliphatischen oder aromatischen Rest,
- der Rest X eine Gruppe $-(CH_2)_n$ und n die Zahl 0 oder 1,
- der Rest Y eine Gruppe $-(CHR^5)_m$ und m die Zahl 0, 1 oder 2 und der Rest $R^5$ Wasserstoff oder eine OH-Gruppe

bedeuten,
mit den Stufen, dass man eine Verbindung der Formel (II)

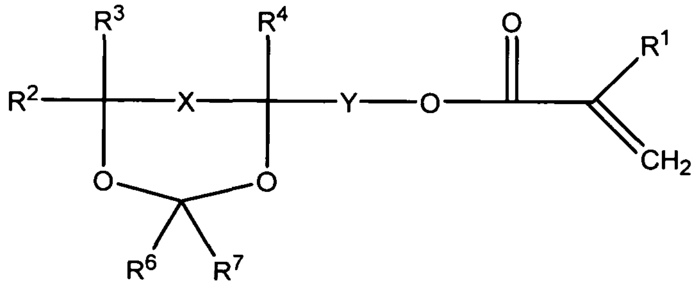

(II)

worin die Reste $R^1$, $R^2$ bis $R^4$, X und Y die oben genannte Bedeutung haben und die Reste $R^6$ und $R^7$ unabhängig voneinander Wasserstoff oder eine aliphatische Gruppe bedeuten,
in wässrigem Medium und in Gegenwart einer in diesem wässrigen Medium gelösten Säure unter Abspaltung einer Verbindung (III)

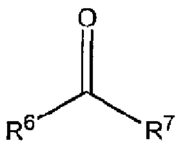

(III)

worin die Reste $R^6$ und $R^7$ die oben genannte Bedeutung haben,
hydrolysiert und die erhaltene Mischung zwecks Neutralisation der in der Mischung vorhandenen Säure mit einer schwachen Base in Kontakt bringt, wobei die Base einen $pK_b$-Wert oberhalb von 3,0 aufweist und in heterogener Form vorliegt.

Die Base muss im Rahmen des erfindungsgemäßen Verfahrens einen $pK_b$-Wert oberhalb von 3,0 aufweisen.

Die Neutralisation der in der Mischung vorhandenen Säure ist ein zentraler Punkt im Rahmen des erfindungsgemäßen Verfahrens. Hierzu wird wie oben ausgeführt eine Base eingesetzt, wobei diese Base einen $pK_b$-Wert oberhalb von 3,0 aufweist und in heterogener Form vorliegt. Unter "in heterogener Form vorliegt" ist zu verstehen, dass die Base nicht in Wasser gelöst vorliegt, sondern in fester Form. In einer bevorzugten Ausführungsform setzt man die Base in immobilisierter Form ein, worunter zu verstehen ist, dass die Base auf der äußeren und/oder inneren Oberfläche von festen wasserunlöslichen Teilchen aufgebracht ist, wobei die Base an diesen Oberflächen physikalisch und/oder chemisch gebunden sein kann. Ganz besonders bevorzugt ist es, als Basen im Rahmen des erfindungsgemäßen Verfahrens basische Ionenaustauscherharze einzusetzen.

In Bezug auf das Merkmal, dass die Base einen $pK_b$-Wert oberhalb von 3,0 aufweisen muß, sei folgendes

ausgeführt. Die Bedeutung von $pK_b$-Werten ist dem Fachmann bekannt. Sie sind ein Maß für die Fähigkeit von Basen, Protonen zu binden und beziehen sich auf folgende Gleichgewichtsreaktion:

$$H_2O\ (1) + B\ (aq) \leftrightarrow BH^+\ (aq) + OH^-\ (aq)$$

In dieser Gleichung bedeutet B eine Base. Die Gleichung beschreibt die Gleichgewichtsreaktion zwischen der Base und Wasser, wobei die Base in dem ihr eigenen Maße Protonen aufnimmt, wobei sie in die protonierte Form $BH^+$ übergeht und wobei logischerweise, da das von der Base aufgenommene Proton aus dem Wasser stammt, dieses in die Form $OH^-$ übergeht. Da $pK_b$-Werte üblicherweise für 25 °C angegeben werden, soll dies auch im Rahmen der vorliegenden Erfindung gelten. Lithiumhydroxid hat einen $pK_b$-Wert von 3,0. Mithin bedeutet die obige Angabe dass die Base im Rahmen des erfindungsgemäßen Verfahrens einen $pK_b$-Wert oberhalb von 3,0 aufweisen muss, dass diese Base schwächer ist als Lithiumhydroxid.

Es sei außerdem auf die dem Fachmann wohlvertraute Tatsache hingewiesen, dass manche Basen mehrere $pK_b$-Werte haben können. Ein Beispiel für eine solche Base ist Bleihydroxid, $Pb(OH)_2$, wofür in der Literatur die Werte $pK_{b1}$ (3,02) und $pK_{b2}$ (7,5) angegeben sind. Ein weiteres Beispiel für eine solche Base ist Ethylendiamin, wofür in der Literatur die Werte $pK_{b1}$ (4,07) und $pK_{b2}$ (7,15) angegeben sind. Analoges gilt etwa für Piperazin, das durch die Werte $pK_{b1}$ (4,19) und $pK_{b2}$ (8,45) charakterisiert ist. Es sei festgestellt, dass die eben genannten Beispiele für Basen mit mehreren $pK_b$-Werten lediglich der Illustration des hier angesprochenen Prinzips dienen sollen.
Der für das erfindungsgemäße Verfahren oben definierte $pK_b$-Wert für die Base (>3,0) bedeutet in Fällen, wo eine Base zum Einsatz kommt, die aufgrund ihrer speziellen Struktur mehr als einen $pK_b$-Wert aufweist, dass dann unter dem $pK_b$-Wert der $pK_{b1}$ - Wert zu verstehen ist.

Wie oben ausgeführt, setzt man im Rahmen des erfindungsgemäßen Verfahrens vorzugsweise basische Ionenaustauscherharze ein. Hierzu ist anzumerken, dass Hersteller solcher Harze typischerweise $pK_a$- Werte für solche Harze angeben. Da der Zusammenhang zwischen $pK_a$ und $pK_a$ wie folgt ist:

$$pK_a + pK_b = 14$$

lässt sich aus einer solchen Angabe in einfacher Weise der $pK_b$-Wert ausrechnen. So hat beispielsweise das Ionenaustauscherharze "Amberlyst A21" laut Hersteller einen $pK_a$-Wert von 8,5, woraus sich unmittelbar ($pK_b = 14 - pK_a$) ein $pK_b$-Wert von 5,5 ergibt. Oder: Aus dem $pK_a$-Wert von 9,5 für "Amberlyst A24"ergibt sich ein $pK_b$-Wert von 4,5.

In einer bevorzugten Ausführungsform des erfindungsgemäßen Verfahrens setzt man ein (schwach) basisches Ionenaustauscherharz ein, das Aminfunktionen enthält, insbesondere tertiäre Aminfunktionen.

Beispiele geeigneter basischer Ionenaustauscherharze sind etwa (1) Amberlite IRA96RF, Amberlyst A21, Amberlyst A23 und Amberlyst A23 von Rohm & Haas sowie (2) Dowex 66, Dowex Monosphere 66, Dowex Monosphere 77, Dowex Marathon WBA, Dowex Marathon WBA-2, Dowex UPCORE Mono WB-500, XUS 43594.00, Dowex M-43 und Dowex M4195 von Dow. Die genannten basischen Ionenaustauscherharze basieren alle - mit Ausnahme von Dowex M4195 - auf tertiären Aminen.

In einer Ausführungsform bedeuten:

- $R^1$ Wasserstoff oder eine Methylgruppe,
- die Reste $R^2$, $R^3$ und $R^4$ Wasserstoff,
- X eine Gruppe-$(CH_2)_n$- und n die Zahl 0,
- Y eine Gruppe -$(CH_2)_m$- und m die Zahl 1 und bedeuten.

In einer weiteren Ausführungsform bedeuten:

- $R^1$ eine Methylgruppe,
- die Reste $R^2$, $R^3$ und $R^4$ Wasserstoff,
- X eine Gruppe-$(CH_2)_n$- und n die Zahl 0,
- Y eine Gruppe -$(CH_2)_m$- und m die Zahl 1 und
- die Reste $R^6$ und $R^7$ jeweils Methylgruppen.

Es ist leicht ersichtlich, dass es sich bei dieser Ausführungsform bei Verbindung (II) um IPGMA, bei Verbindung (III) um Aceton und bei Verbindung (I) um GMMA handelt.

Als Säure setzt man in der Hydrolysestufe des erfindungsgemäßen Verfahrens vorzugsweise eine Mineralsäure ein, insbesondere eine, die ausgewählt ist aus der Gruppe Schwefelsäure, Salzsäure und Bromwasserstoffsäure.

In einer Ausführungsform erfindungsgemäßen Verfahrens leitet man während der Hydrolyse einen Gasstrom durch die Reaktionsmischung, um die freigesetzte Verbindung (III) aus dem Reaktionsgemisch zu entfernen. Das dabei eingesetzte Gas ist vorzugsweise Luft. Hierbei führt man die Hydrolyse vorzugsweise durch kontinuierliche Zufuhr von Wasser bei konstantem Volumen durch.

In einer Ausführungsform erfindungsgemäßen Verfahrens hydrolysiert man die Verbindung (II) in Gegenwart eines Inhibitors. Die Art des Inhibitors ist an sich nicht kritisch. Vorzugsweise setzt man als Inhibitor Hydochinonmonomethylether (MEHQ) ein.

In einer bevorzugten Ausführungsform des erfindungsgemäßen Verfahrens führt man die Hydrolyse in Abwesenheit organischer Lösungsmittel durch.

In einer Ausführungsform des erfindungsgemäßen Verfahrens filtriert man nach der Neutralisation. Dabei ist es bevorzugt, die bei der Filtration erhaltene wässrige Lösung von (I) durch Abstrippen von Wasser zu befreien, wobei man zum Abstrippen insbesondere Filmverdampfer (film evaporators) oder Röhrenverdampfer (tube evaporators) einsetzt. Vorzugsweise setzt man Filmverdampfer vom Typ Dünnschichtverdampfer (thin-film evaporators) ein, zum Beispiel Rotorverdampfer (rotary evaporators) oder Dünnschichtverdampfer mit Wischblättern (wiped film evaporators). Vorzugsweise setzt man Röhrenverdampfer vom Typ Fallfilmverdampfer (falling film evaporators) oder Steigrohrverdampfer (rising film evaporators) ein.

Die Neutralisationsstufe des erfindungsgemäßen Verfahrens kann diskontinuierlich (batchweise) oder kontinuierlich durchgeführt werden.

Eine weitere Offenbarung ist die Verwendung der Monomeren (I), erhältlich nach dem erfindungsgemäßen Verfahren, zur Herstellung von Polymeren, insbesondere von Polymeren, die Einsatz im medizinischen Bereich finden, beispielsweise für Geräte und Vorrichtungen im Bereich der Ophthalmologie, Implantate, Prothesen und Hydrogel-Artikeln. Die nach dem erfindungsgemäßen Verfahren erhältlichen Monomeren (I) werden vorzugsweise zur Herstellung von Kontaktlinsen verwendet, wobei sie als alleinige Monomere oder in Kombination mit weiteren Comonomeren eingesetzt werden können. Unter Kontaktlinsen sind dabei sowohl harte als auch weiche Kontaktlinsen zu verstehen.

Eine weitere Offenbarung ist ein Verfahren zur Herstellung von Polymeren auf Basis von ein oder mehreren Monomeren (I), erhältlich nach dem erfindungsgemäßen Verfahren und gegebenenfalls zusätzlich ein oder mehreren Comonomeren. Unter Polymeren sind dabei vorzugsweise solche zu verstehen, die Einsatz im medizinischen Bereich finden, beispielsweise für Geräte und Vorrichtungen im Bereich der Ophthalmologie, Implantate, Prothesen und Hydrogel-Artikeln und insbesondere harte oder weiche Kontaktlinsen. Die Herstellung derartiger Polymere kann im Prinzip nach allem den Fachmann dafür einschlägig bekannten Methoden erfolgen. Es können dabei auch alle üblichen Hilfsmittel und Additive eingesetzt werden. Beispiele geeigneter Additive sind die in EP-B-1,171410, Seite 7, Zeilen 8-12 beschrieben. Beispiele geeigneter Tenside und Dispergiermittel sind die in EP-B-1,171410, Seite 7, Zeilen 13-20 beschrieben. Beispiele geeigneter Comonomerer sind die in EP-B-1,171410, Seite 7, Zeilen 26 - Seite 8, Zeile 16 beschrieben.

Ein weiterer Erfindungsgegenstand ist ein Verfahren zur Herstellung von Kontaktlinsen, bei dem

(i) polymerisierbare Monomere der Formel (I) nach dem erfindungsgemäßen Verfahren hergestellt werden und
(ii) die in Schritt (i) erhaltenen polymerisierbaren Monomere der Formel (I) als alleinige Monomere oder in Kombination mit weiteren Comonomeren eingesetzt werden, um die Kontaktlinsen zu erhalten.

## Beispiele

Beispiel 1

27 kg Isopropylidenglycerinmethacrylat (IPGMA) mit einem Gehalt von 80 ppm MEHQ (dieses IPGMA war durch Umesterung von Methylmethacrylat und 2,2-Dimethyl-4-hydroxymethyl-1,3-dioxolan hergestellt worden) und 17,8 kg of demineralisiertem Wasser wurden in einen Reaktor gefüllt, der mit einem Lufteinleitungsrohr ausgerüstet war. Die Mischung wurde gerührt, wobei ein Luftstrom (10 Liter pro Minute) durch die Reaktionsmischung geleitet wurde.

77g einer 10 Gew.-%-igen wäßrigen Salzsäure wurden zudosiert und die Reaktion bei 20 °C weitergeführt. Der durch den Luftstrom verursachte Wasserverlust im Reaktor wurde durch permanentes Zudosieren von Wasser ausgeglichen, so dass die Reaktion bei konstantem Volumen durchgeführt wurde. Die Reaktion wurde solange fortgesetzt, bis der Gehalt der Reaktionsmischung an IPGMA unterhalb von 0,16 Gew.-% lag. Die hierfür benötigte Reaktionszeit betrug 48 Stunden.

Die Reaktionsmischung wurde dann zur Entfernung überschüssiger Säure durch Zufügen von 1,9 kg Amberlyst A24 (handelsübliches basisches Ionenaustauscherharz von Rohm & Haas) neutralisiert, wobei dieses Ionenaustauscherharz vorher mit deionisiertem Wasser frisch gewaschen worden war. Die Aufschlämmung wurde 1 Stunde gerührt, wonach die Acidität unterhalb von 0,05 mg KOH/g betrug. Danach wurde die Aufschlämmung filtriert, wobei eine wässrige Lösung von GMMA erhalten wurde.

Aus dieser wässrigen Lösung von GMMA wurde das darin vorhandene Wasser durch Abstrippen mittels eines

Dünnschichtverdampfers mit Wischblättern (wiped film evaporator) (ein Standard 1/100 System der Firma Lab-Plant Distillation Systems) entfernt. Die Hauptsäule hatte einen Innendurchmesser von 100 mm, die beheizte Länge betrug 480 mm, die beheizte Oberfläche betrug 0,15m$^2$. Die wässrige Lösung von GMMA wurde der Destillationseinheit über einen Vorheizer, der auf eine Tempatur von 40 °C eingestellt war, in einer Menge von 800 g/h zugeführt. Die Wände des Verdampfers waren auf eine Temperatur von 60 °C beheizt. Die wässrige GMMA Lösung wurde in 3 Durchgängen abgestrippt, wobei das Vakuum von 50 mbar auf 10 mbar gesenkt wurde. Auf diese Weise wurde ein GMMA hoher Reinheit erhalten, das wie folgt charakterisiert wurde:

- Wassergehalt unterhalb von 1 Gew.-%
- Gehalt an Glycerin-di-methacrylat (GDMA) unterhalb von 0,06 Gew.-%
- Gehalt an Methacrylsäure unterhalb von 0,02 Gew.-%

**Patentansprüche**

**1.** Verfahren zur Herstellung von polymerisierbaren Monomeren der Formel (I)

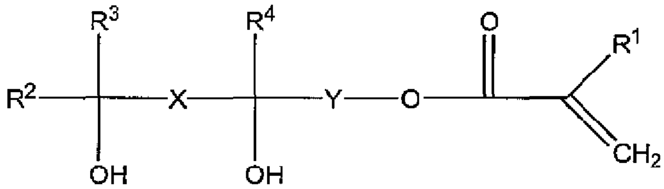

(I)

worin

- der Rest $R^1$ Wasserstoff oder eine Alkylgruppe mit 1 bis 4 C-Atomen,
- die Reste $R^2$ bis $R^4$ unabhängig voneinander Wasserstoff oder einen aliphatischen, cycloaliphatischen oder aromatischen Rest,
- der Rest X eine Gruppe $-(CH_2)_n-$ und n die Zahl 0 oder 1,
- der Rest Y eine Gruppe $-(CHR^5)_m-$ und m die Zahl 0, 1 oder 2 und der Rest $R^5$ Was-serstoff oder eine OH-Gruppe

bedeuten,
mit den Stufen, dass man eine Verbindung der Formel (II)

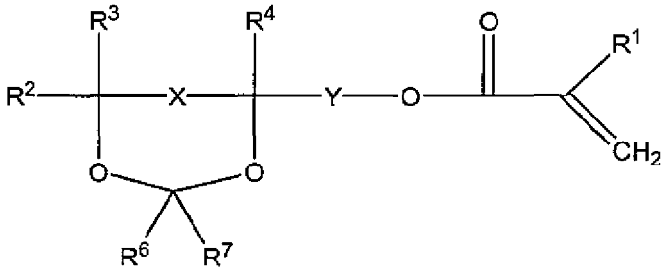

(II)

worin die Reste $R^1$, $R^2$ bis $R^4$, X und Y die oben genannte Bedeutung haben und die Reste $R^6$ und $R^7$ unabhängig voneinander Wasserstoff oder eine aliphatische Gruppe bedeuten,
in wässrigem Medium und in Gegenwart einer in diesem wässrigen Medium gelösten Säure unter Abspaltung einer Verbindung (III)

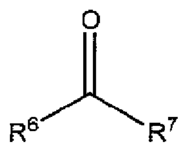

(III)

worin die Reste $R^6$ und $R^7$ die oben genannte Bedeutung haben, hydrolysiert und die erhaltene Mischung zwecks Neutralisation der in der Mischung vorhandenen Säure mit einer schwachen Base in Kontakt bringt, wobei die Base einen $pK_b$-Wert oberhalb von 3,0 aufweist und in heterogener Form vorliegt.

2. Verfahren nach Anspruch 1, wobei

- $R^1$ Wasserstoff oder eine Methylgruppe,
- die Reste $R^2$, $R^3$ und $R^4$ Wasserstoff,
- X eine Gruppe $-(CH_2)_n-$ und n die Zahl 0,
- Y eine Gruppe $-(CH_2)_m-$ und m die Zahl 1

bedeuten.

3. Verfahren nach Anspruch 2, wobei

- der Rest $R^1$ eine Methylgruppe und
- die Reste $R^6$ und $R^7$ jeweils Methylgruppen

bedeuten.

4. Verfahren nach einem der Ansprüche 1 bis 3, wobei man als Säure eine Mineralsäure einsetzt und wobei man die Mineralsäure auswählt aus der Gruppe Schwefelsäure, Salzsäure und Bromwasserstoffsäure.

5. Verfahren nach einem der Ansprüche 1 bis 4, wobei man als immobilisierte Base ein basisches Ionenaustauscherharz einsetzt, das Aminfunktionen enthält.

6. Verfahren nach einem der Ansprüche 1 bis 5, wobei man während der Hydrolyse einen Gasstrom durch die Reaktionsmischung leitet, um die freigesetzte Verbindung (III) aus dem Reaktionsgemisch zu entfernen.

7. Verfahren nach einem der Ansprüche 1 bis 6, wobei man die Verbindung (II) in Gegenwart eines Inhibitors hydrolysiert.

8. Verfahren nach einem der Ansprüche 1 bis 7, wobei man die Hydrolyse in Abwesenheit organischer Lösungsmittel durchführt.

9. Verfahren nach einem der Ansprüche 1 bis 8, wobei man nach der Neutralisation filtriert.

10. Verfahren nach Anspruch 9, wobei man die bei der Filtration erhaltene wässrige Lösung von (I) durch Abstrippen von Wasser befreit.

11. Verfahren nach einem der Ansprüche 1 bis 10, wobei man die Neutralisation diskontinuierlich durchführt.

12. Verfahren nach einem der Ansprüche 1 bis 10, wobei man die Neutralisation kontinuierlich durchführt.

13. Verfahren zur Herstellung von Kontaktlinsen, bei dem

(i) polymerisierbare Monomere der Formel (I) nach einem Verfahren gemäß einem der Ansprüche 1 bis 12 hergestellt werden und
(ii) die in Schritt (i) erhaltenen polymerisierbaren Monomere der Formel (I) als alleinige Monomere oder in

Kombination mit weiteren Comonomeren eingesetzt werden, um die Kontaktlinsen zu erhalten.

## Claims

**1.** A process for the production of polymerizable monomers corresponding to formula (I):

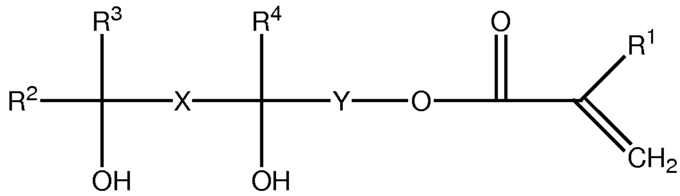

(I)

in which

- the substituent $R^1$ is hydrogen or a $C_{1-4}$ alkyl group,
- the substituents $R^2$ to $R^4$ independently of one another represent hydrogen or an aliphatic, cycloaliphatic or aromatic radical,
- the substituent X is a group $-(CH_2)_n-$ and n is the number 0 or 1,
- the substituent Y is a group $(CHR^5)_m-$ and m is the number 0, 1 or 2 and the substituent $R^5$ is hydrogen or an OH group

comprising the steps of hydrolyzing a compound corresponding to formula (II):

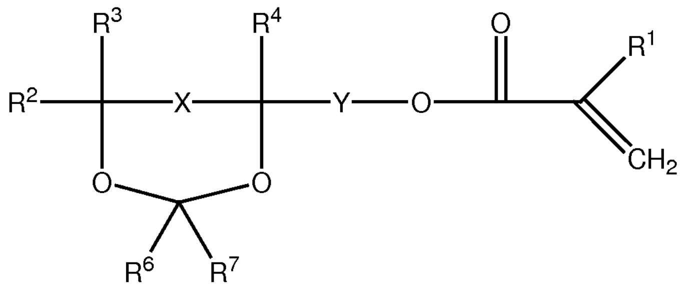

(II)

in which the substituents $R^1$, $R^2$ to $R^4$, X and Y are as defined above and the substituents $R^6$ and $R^7$ independently of one another represent hydrogen or an aliphatic group,
in aqueous medium and in the presence of an acid dissolved in said aqueous medium, the hydrolysis being accompanied by elimination of a compound (III):

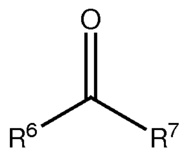

(III)

in which the substituents $R^6$ and $R^7$ are as defined above,
and contacting the mixture obtained with a weak base to neutralize the acid present in the mixture, the base having a $pK_b$ value above 3.0 and being present in heterogeneous form.

**2.** A process as claimed in claim 1, wherein

- $R^1$ is hydrogen or a methyl group,
- the substituents $R^2$, $R^3$ and $R^4$ represent hydrogen,
- X is a group $-(CH_2)_n-$ and n is the number 0,
- Y is a group $-(CH_2)_m-$ and m is the number 1.

**3.** A process as claimed in claim 2, wherein

- the substituent $R^1$ is a methyl group and
- the substituents $R^6$ and $R^7$ are each methyl groups.

**4.** A process as claimed in any of claims 1 to 3, wherein a mineral acid is used and wherein said mineral acid is selected from the group consisting of sulfuric acid, hydrochloric acid and hydrobromic acid.

**5.** A process as claimed in any of claims 1 to 4, wherein a basic ion exchange resin which contains amine functions is used as the immobilized base.

**6.** A process as claimed in any of claims 1 to 5, wherein a gas stream is passed through the reaction mixture during the hydrolysis step to remove the compound (III) released from the reaction mixture.

**7.** A process as claimed in any of claims 1 to 6, wherein the compound (II) is hydrolyzed in the presence of an inhibitor.

**8.** A process as claimed in any of claims 1 to 7, wherein the hydrolysis is carried out in the absence of organic solvents.

**9.** A process as claimed in any of claims 1 to 8, wherein the neutralization step is followed by filtration.

**10.** A process as claimed in claim 9, wherein the aqueous solution of (I) obtained after filtration is freed from water by stripping.

**11.** A process as claimed in any of claims 1 to 10, wherein the neutralization step is carried out discontinuously.

**12.** A process as claimed in any of claims 1 to 10, wherein the neutralization step is carried out continuously.

**13.** A process for the production of contact lenses, in which

(i) polymerizable monomers of the formula (I) are produced by a process as claimed in any of claims 1 to 12 and
(ii) the polymerizable monomers of the formula (I) obtained in step (i) are used as sole monomer or in combination with other comonomers, in order to obtain the contact lenses.

## Revendications

**1.** Procédé de fabrication de monomères polymérisables de formule (I)

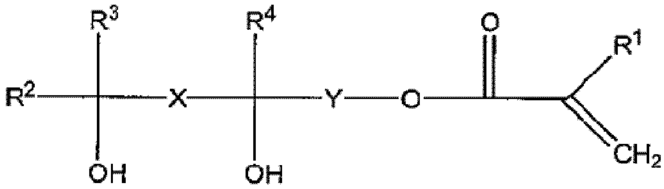

(I)

dans laquelle

- le radical $R^1$ signifie l'hydrogène ou un groupe alkyle de 1 à 4 atomes C,
- les radicaux $R^2$ à $R^4$ signifient indépendamment les uns des autres l'hydrogène ou un radical aliphatique, cycloaliphatique ou aromatique,
- le radical X signifie un groupe $-(CH_2)_n-$ et n le nombre 0 ou 1,

- le radical Y signifie un groupe - $(CHR^5)_m$- et m le nombre 0, 1 ou 2, et le radical $R^5$ signifie l'hydrogène ou un groupe OH,

comprenant les étapes selon lesquelles un composé de formule (II)

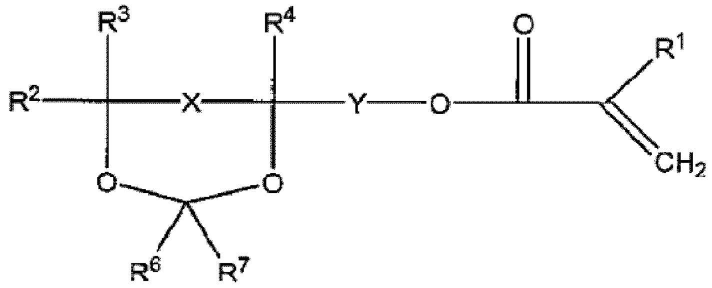

(II)

dans laquelle les radicaux $R^1$, $R^2$ à $R^4$, X et Y ont la signification indiquée précédemment, et les radicaux $R^6$ et $R^7$ signifient indépendamment l'un de l'autre l'hydrogène ou un groupe aliphatique,
est hydrolysé dans un milieu aqueux et en présence d'un acide dissous dans ce milieu aqueux avec clivage d'un composé (III)

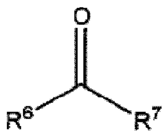

(III)

dans laquelle les radicaux $R^6$ et $R^7$ ont la signification indiquée précédemment,
et le mélange obtenu est mis en contact avec une base faible pour la neutralisation de l'acide présent dans le mélange, la base présentant une valeur de $pK_b$ supérieure à 3,0 et se présentant sous forme hétérogène.

**2.** Procédé selon la revendication 1, dans lequel

- $R^1$ signifie l'hydrogène ou un groupe méthyle,
- les radicaux $R^2$, $R^3$ et $R^4$ signifient l'hydrogène,
- X signifie un groupe $-(CH_2)_n-$ et n le nombre 0,
- Y signifie un groupe $-(CH_2)_m-$ et m le nombre 1.

**3.** Procédé selon la revendication 2, dans lequel

- le radical $R^1$ signifie un groupe méthyle et
- les radicaux $R^6$ et $R^7$ signifient chacun des groupes méthyle.

**4.** Procédé selon l'une quelconque des revendications 1 à 3, dans lequel un acide minéral est utilisé en tant qu'acide et dans lequel l'acide minéral est choisi dans le groupe constitué par l'acide sulfurique, l'acide chlorhydrique et l'acide bromhydrique.

**5.** Procédé selon l'une quelconque des revendications 1 à 4, dans lequel une résine échangeuse d'ions basique qui contient des fonctions amine est utilisée en tant que base immobilisée.

**6.** Procédé selon l'une quelconque des revendications 1 à 5, dans lequel un courant gazeux est passé au travers du mélange réactionnel pendant l'hydrolyse pour éliminer le composé (III) libéré du mélange réactionnel.

**7.** Procédé selon l'une quelconque des revendications 1 à 6, dans lequel le composé (II) est hydrolysé en présence d'un inhibiteur.

**8.** Procédé selon l'une quelconque des revendications 1 à 7, dans lequel l'hydrolyse est réalisée en l'absence de solvants organiques.

**9.** Procédé selon l'une quelconque des revendications 1 à 8, dans lequel une filtration est effectuée après la neutralisation.

**10.** Procédé selon la revendication 9, dans lequel la solution aqueuse de (I) obtenue lors de la filtration est débarrassée de l'eau par extraction.

**11.** Procédé selon l'une quelconque des revendications 1 à 10, dans lequel la neutralisation est réalisée de manière discontinue.

**12.** Procédé selon l'une quelconque des revendications 1 à 10, dans lequel la neutralisation est réalisée de manière continue.

**13.** Procédé de fabrication de lentilles de contact, selon lequel

(i) des monomères polymérisables de formule (I) sont fabriqués par un procédé selon l'une quelconque des revendications 1 à 12, et
(ii) les monomères polymérisables de formule (I) obtenus à l'étape (i) sont utilisés en tant que monomères uniques ou en combinaison avec d'autres comonomères pour obtenir les lentilles de contact.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 852384 A
- US 4056496 A
- US 6011081 A
- EP 1171410 B
- EP 1171411 B
- WO 0063149 A
- WO 0063150 A
- DE 10349972 A